# EUROPÄISCHE PATENTSCHRIFT

(11) **EP 2 421 455 B1**
(45) Veröffentlichungstag und Bekanntmachung des Hinweises auf die Patenterteilung: **21.09.2016**
(21) Anmeldenummer: 10717486.4
(22) Anmeldetag: 19.04.2010
(51) Int. Cl.: A61B 17/80

(54) **BEFESTIGUNGSVORRICHTUNG FÜR CHIRURGISCHE HALTESYSTEME**
FASTENING APPARATUS FOR SURGICAL RETAINING SYSTEMS
DISPOSITIF DE FIXATION POUR SYSTÈMES DE MAINTIEN CHIRURGICAUX

(30) Priorität: 20.04.2009 CH 6192009
(43) Veröffentlichungstag der Anmeldung: 29.02.2012
(73) Patentinhaber: Creaholic SA, 2503 Biel (CH)
(72) Erfinder: RUSCH, Christoph, CH-2502 Biel (CH)
(74) Vertreter: Frei Patent Attorneys
(86) Internationale Anmeldenummer: PCT/CH2010/000105
(87) Internationale Veröffentlichungsnummer: WO 2010/121388

(56) Entgegenhaltungen:
- EP-A2- 0 988 833
- EP-A2- 1 346 697
- FR-A1- 2 792 185
- US-A1- 2004 122 426
- US-A1- 2005 149 027

## Beschreibung

Die Erfindung bezieht sich auf das Gebiet der chirurgischen Haltesysteme und insbesondere auf eine Befestigungsvorrichtung für chirurgische Haltesysteme, und ein chirurgisches Halteelement, gemäss dem Oberbegriff der entsprechenden unabhängigen Patentansprüche.

### STAND DER TECHNIK

Chirurgische Haltesysteme, beispielsweise zum Fixieren von gebrochenen Knochen bestehen oft aus Platten, welche mittels Schrauben in den Knochen befestigt sind. Die Platten weisen Öffnungen auf, in welche die Schrauben unter verschiedenen Winkeln eingebracht werden. Während des Einschraubens der Schrauben in den Knochen können die Schrauben in der Platte fixiert werden, beispielsweise durch ein Gewinde am Schraubenkopf, welches in die Platte einschneidet.

Nachteil dieser Ausführung ist, dass die Haltesysteme nach dem Festschrauben verspannt sein können, und durch die bereits befestigten Schrauben die Position einer Platte nicht mehr korrigierbar ist. Ein Nachrichten der Platte, durch Lösen und wieder Anziehen der Schrauben, bedingt unter Umständen ein Ersetzen der Befestigungsschrauben und/oder der Platte. Ein weiterer Nachteil ist, dass die Schrauben die Platte gegen den Knochen drücken. Dies kann zu einem Weichen des Knochens und dadurch zu einer losen Verbindung führen.

EP 1 346 697 A2 zeigt eine Befestigungsvorrichtung für chirurgische Haltesysteme, aufweisend eine Schraube, welche in einer Platte fixierbar ist. Die Schraube wird gemäss verschiedenen Ausführungsformen blockiert, indem der Kopf abgedeckt wird, wobei auch ein Spielraum verbleiben kann. Dazu können Ringe mit exzentrischer Öffnung vorliegen. Damit geschieht das Blockieren der Schrauben, indem durch Verdrehen eines exzentrischen Ringes die Öffnung verschoben wird, durch welche die Schraube herausdrehen könnte. Eine derart abgedeckte Schraube kann sich aber immer noch um ihre Achse drehen, es ist also kein vollständiges Fixieren der Schraube möglich.

FR 2 792 185 und EP 0 988 833 A2 zeigen das Arretieren eines sphärischer Schraubenkopfes, indem ein Teil mit einer Innenkugelfläche als Klemmelement gegen einen kugelförmigen Schraubenkopf geschraubt wird. Die Achse dieser Schraubbewegung führt zwingend durch den Mittelpunkt der Kugelfläche des Schraubenkopfes. Es sind also mehrere Umdrehungen zum Einschrauben des Klemmelementes erforderlich, was nachteilig ist.

### DARSTELLUNG DER ERFINDUNG

Es ist deshalb Aufgabe der Erfindung, eine Befestigungsvorrichtung für chirurgische Haltesysteme und ein chirurgisches Halteelement der eingangs genannten Art zu schaffen, welche die oben genannten Nachteile beheben.

Diese Aufgabe lösen eine Befestigungsvorrichtung für chirurgische Haltesysteme und ein chirurgisches Halteelement mit den Merkmales der entsprechenden unabhängigen Patentansprüche.

Die Befestigungsvorrichtung, vorgesehen für chirurgische Haltesysteme weist mindestens ein Halteelement und ein Befestigungselement auf, welche mechanisch miteinander verbunden werden können. Das Halteelement weist eine Gelenkpfanne auf, und das Befestigungselement einen Gelenkkopf. Die Gelenkpfanne und der Gelenkkopf korrespondieren vorzugsweise zumindest abschnittsweise in ihrer form miteinander und bilden zusammen ein Kugelgelenk. Die Bewegung des Kugelgelenkes ist mittels eines Klemmelementes arretierbar.

Das Kugelgelenk kann dadurch realisiert sein, dass sowohl der Gelenkkopf als auch die Gelenkpfanne gänzlich zueinanderpassende sphärische Flächen aufweisen. In einer zweiten bevorzugten Ausführungsform der Erfindung ist die Gelenkpfanne nur in einem Teilbereich sphärisch, und in einem übrigen Bereich sich gegen das Klemmelement hin öffnend, vorzugsweise konisch, ausgebildet. Der Teilbereich liegt dabei an der Seite des Haltelementes, welche dem Klemmelement gegenüber liegt, der übrige Bereich liegt zwischen dem Teilbereich und dem Klemmelement. In einer dritten bevorzugten Ausführungsform der Erfindung ist die Gelenkpfanne vollständig sich gegen das Klemmelement hin öffnend, vorzugsweise konisch, ausgebildet. Auch in der zweiten und der dritten Ausführungsform der Erfindung wird aufgrund der sphärischen Form des Gelenkkopfes mit der Gelenkpfanne eine Kugelgelenkverbindung gebildet.

Dadurch wird es möglich, eine chirurgische Platte bzw. ein Halteelement in seiner Position zu justieren, auch wenn eines oder mehrere Befestigungselemente bereits fest mit einem Substrat, beispielsweise mit einem Knochen, verbunden sind. Dies wird erreicht, indem die Positionierung des Halteelementes durch das Klemmelement gelöst und wieder verriegelt wird. Ferner ist es möglich, die Schrauben in einem relativ frei wählbaren Winkel in den Knochen zu schrauben, und unabhängig von der genauen Position der Schrauben diese mit der Platte winkelstabil zu verriegeln. Dabei ist es nicht nötig, dass die Schraube die Platte gegen den Knochen presst.

Ein weiterer Vorteil ist, dass die Arretierung des Befestigungselementes bezüglich des Halteelementes unabhängig davon geschehen kann, ob das Halteelemement gegen das Substrat gedrückt wird oder nicht. So kann beispielsweise, bei gelöstem Klemmelement, durch Drehen eines mit einem Schraubengewinde versehenen Befestigungselementes ein Knochen mehr oder weniger weit gegen das Haltelement gezogen werden. Dies kann notwendig sein, um den Knochen auf eine vorgegebene Distanz bezüglich des Halteelements zu positionieren, oder um nach einer gewissen Zeit die Verbindung nachzuspannen. Im Gegensatz zum Stand der Technik, wo die Arretierung der Schraube erst beim Anpressen der Schraube gegen eine Platte als Halteelements geschieht, liegt bei der Erfindung also eine grössere Freiheit in der Positionierung des Halteelements vor.

Das Kugelgelenk der Befestigungsvorrichtung lässt im arretierten Zustand mittels des Klemmelementes keine translatorische Bewegung des Gelenkkopfes bezüglich der Gelenkpfanne zu. Insbesondere ist also das Halteelement nicht entlang des Befestigungselements verschiebbar, wie das bei einer einfachen Schraube der Fall wäre. Die Gelenkpfanne umschliesst dazu vorzugsweise den Gelenkkopf des Befestigungselementes mindestens teilweise und zentriert den Gelenkkopf bzw. das Kugelzentrum innerhalb des Halteelementes. Dabei ist der Gelenkkopf vorzugsweise fast vollständig im Halteelement versenkbar.

Das Klemmelement der Befestigungsvorrichtung weist einen Ring mit exzentrischer Form auf, welcher beim Verdrehen des Ringes den Gelenkkopf bezüglich der Gelenkpfanne verklemmt. Eine innere sowie eine äussere Fläche des Ringes weisen vorzugsweise eine rotationssymmetrische Form auf, wobei die innere und äussere Fläche des Ringes vorzugsweise exzentrisch zueinander verlaufen und somit die Achsen der inneren sowie der äusseren Ringflächen sich nicht überdecken oder nicht aufeinander fallen.

Das Halteelement weist vorzugsweise eine umlaufende Nut, insbesondere eine Ringnut auf, welche an die Gelenkpfanne anschliesst. Die Innenfläche der umlaufenden Nut verläuft vorzugsweise rotationssymmetrisch, aber exzentrisch bezüglich der Gelenkpfanne und ist korrespondierend zur Aussenfläche des Ringes geformt. Durch die exzentrisch angelegte Form der Ringnut und des Ringes wird es möglich, den Gelenkkopf durch Drehen des Ringes in der Gelenkpfanne zu verspannen.

Das Klemmelement ist mit einer Kontaktfläche versehen, die vorzugsweise eine zum Gelenkkopf korrespondierende Geometrie aufweist. Beim Verdrehen des Klemmelementes drückt die Kontaktfläche gegen die kugelabschnittsförmige Aussenfläche des Gelenkkopfes. Die Aussenseite des Klemmelementes weist vorzugsweise einen Absatz und/oder einen Konus auf der mit der Form der Ringnut korrespondiert. Das Klemmelement weist vorzugsweise eine oder mehrere Führungen auf, die für den Eingriff eines Werkzeuges geeignet sind, um eine Verklemmungsbewegung vorzunehmen.

In einer bevorzugten Ausfiihrungsform der Erfindung weist das Klemmelement eine Öffnung auf, welche ein Zusammendrücken des Klemmelementes in Durchmesserrichtung erlaubt. Diese Öffnung kann gleichzeitig auch als Führung für das Werkzeug dienen. Eine weitere Führung im Befestigungselementes ist beispielsweise eine U-förmige Aussparung. Diese bewirkt zudem eine biegeschwache Stelle, die das Klemmelement biegbar macht. Diese biegeschwache Stelle ermöglicht ein vereinfachtes Hineindrücken des Klemmelementes in die dafür vorgesehene Ringnut am Halteelement.

In einer weiteren bevorzugten Ausführungsform weist das Klemmelement Nocken für einen Bajonettverschluss auf, die in radialer Richtung herausragen. Korrespondierend sind dann am Halteelement Einschnitte in Form von radialen Schlitzen gebildet, die zum Einführen der Nocken des Klemmelementes vorgesehen sind. Die Verbindung zwischen dem Klemmelement und dem Halteelement kann somit durch eine Steck-Dreh-Bewegung erfolgen, ohne dass das Klemmelement zusammengedrückt werden muss.

Die Kontaktfläche des Klemmelementes entspricht mindestens annähernd einem Abschnitt der Geometrie eines Gelenkkopfes, wobei die Kontaktfläche des Klemmelementes und/oder der Gelenkpfanne und/oder der Gelenkkopf vorzugsweise eines oder mehrere Fixierelemente aufweist, welche über den restlichen Bereich der Kontaktfläche vorstehen. Durch Verdrehen des Klemmelementes können sich diese Fixierelemente in das korrespondierende Teil einschneiden. Die Fixierelemente können beispielsweise einzelne oder mehrere parallel zur Ebene des Klemmelementes umlaufende Schneidkanten aufweisen, die selber wiederum mehrere einzelne Abschnitte aufweisen können. Die Fixierelemente können auch mikroskopisch ausgebildet sein, indem eine oder mehrere der in Kontakt stehenden Flächen an Klemmelement und/oder Gelenkpfanne und/oder Gelenkkopf aufgerauht sind. In einer bevorzugten Ausführungsform der Erfindung sind die Fixierelemente an der Gelenkpfanne und/oder an einem unteren Teil des Gelenkkopfes ausgebildet, der gegen die Gelenkpfanne pressbar ist. Dies hindert den Gelenkkopf daran, sich beim Anziehen des Klemmelementes mitzudrehen.

Das Befestigungselement weist vorzugsweise einen Schaft auf, der optional ein Gewinde oder eine andere Formschlussverbindung oder Kraftschlussverbindung enthalten kann. In anderen Ausführungsformen der Erfindung ist der Schaft für eine stoffschlüssige Verbindung vorgesehen. Der Gelenkkopf des Befestigungselementes ist vorzugsweise mit einer Aufnahme für ein Werkzeug versehen. In einer bevorzugten Ausfiihrungsform des Erfindungsgegenstandes ist die Aufnahme des Befestigungselementes so geformt, dass ein Werkzeug in einem - in Grenzen - frei beweglichem Winkel zu der Längsachse des Befestigungselementes in die Aufnahme eingebracht werden kann. Eine Rotationsachse des Befestigungselementes verläuft vorzugsweise durch das Zentrum des Gelenkkopfes.

An dem chirurgischen Halteelement sind eine oder mehrere Gelenkpfannen mit korrespondierenden Ringnuten ausgebildet. Das Halteelement kann schon vor der Verbindung mit einem Befestigungselement mit einem oder mehreren Ringen bestückt sein.

Im Befestigungsverfahren wird vorzugsweise das Befestigungselement in die Gelenkpfanne des Halteelementes gesteckt, wo die Ringe oder Klemmelemente beispielsweise bereits vorinstalliert sind. Nach dem groben Ausrichten des Halteelementes in die gewünschte Position werden die Befestigungselemente vorzugsweise durch das Haltelelement hindurch mit dem Substrat, beispielsweise mit einem Knochen festgeschraubt. Die Klemmelemente sind noch nicht im verriegeltem Zustand, so dass das Halteelement in seiner Orientierung zum Substrat spannungsfrei positioniert werden kann. Die Befestigungselemente werden durch ein Verdrehen der Klemmelemente mittels eines Werkzeuges mit dem Halteelement verriegelt. Nachkorrekturen können durch Lösen von einzelnen oder mehreren Klemmelementen ausgeführt werden. Das Klemmelement kann mittels eines rohrartigen Schlüssels während der Installation des Befestigungselementes in seiner Position sowie während dem Befestigen oder einem späterem Verstellen des Befestigungselementes gehalten werden, und dadurch gegen Mitdrehen mit dem Befestigungselement gesichert werden. Dadurch wird ein unerwünschtes Verklemmen des Klemmelementes verhindert.

Grundsätzlich kann die beschriebene Befestigungsvorrichtung auch in anderen Anwendungen eingesetzt werden, beispielsweise im Maschinenbau, für Halterungen und Stative und dergleichen.

Weitere bevorzugte Ausführungsformen gehen aus den abhängigen Patentansprüchen hervor. Dabei sind Merkmale der Verfahrensansprüche sinngemäss mit den Vorrichtungsansprüchen kombinierbar und umgekehrt.

### KURZE BESCHREIBUNG DER ZEICHNUNGEN

Im folgenden wird der Erfindungsgegenstand anhand von bevorzugten Ausführungsbeispielen, welche in den beiliegenden Zeichnungen dargestellt sind, näher erläutert. Es zeigen jeweils schematisch:
- Figur 1: eine Befestigungsvorrichtung in nicht arretiertem Zustand;
- Figur 2: eine Explosionszeichnung der Befestigungsvorrichtung;
- Figur 3: einen Schnitt durch die Befestigungsvorrichtung im nicht arretierten Zustand;
- Figur 4: einen Schnitt durch die Befestigungsvorrichtung in einem arretierten Zustand;
- Figur 5: die Befestigungsvorrichtung in arretiertem Zustand;
- Figur 6: eine Befestigungsvorrichtung in arretiertem Zustand in einer weiteren Ausführungsform; und
- Figur 7: verschiedene Formen von Gelenkpfannen.

Die in den Zeichnungen verwendeten Bezugszeichen und deren Bedeutung sind in der Bezugszeichenliste zusammengefasst aufgelistet. Grundsätzlich sind in den Figuren gleiche Teile mit gleichen Bezugszeichen versehen.

### WEGE ZUR AUSFÜHRUNG DER ERFINDUNG

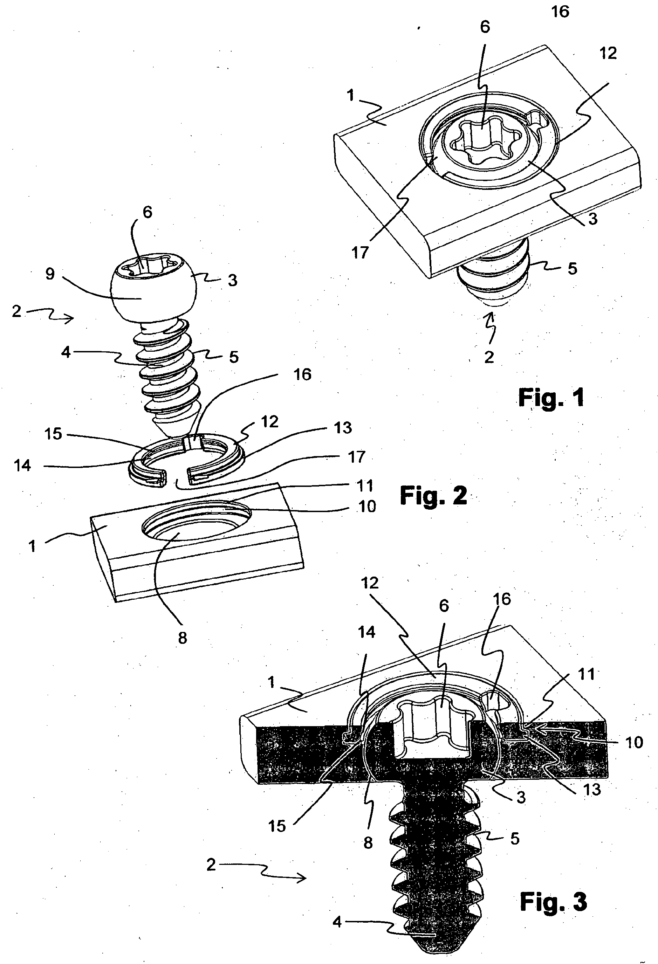

**Figur 1** zeigt eine Befestigungsvorrichtung mit einem Halteelement 1, einem Befestigungselement 2 sowie einem Klemmelement 12 im nicht arretierten Zustand. **Figur 2** zeigt die verschiedenen Elemente in einer Explosionszeichnung. Das Befestigungselement 2 mit einem Gelenkkopf 3 ist in einem Halteelement 1, das eine Aufnahme für das Befestigungselement 2 mit einer Gelenkpfanne 8 aufweist, eingelegt. Das Befestigungselement 2 weist eine Aufnahme für Steckschlüssel 6 sowie einen Schaft 4 auf. Der Schaft 4 ist beispielsweise mit einem Gewinde 5 versehen und kann in ein zu fixierendes oder stabilisierendes Substrat, beispielsweise in einen Knochen, eingeschraubt werden. Durch das Kugelgelenk aus Gelenkpfanne 8 und Gelenkkopf 3 ist das Befestigungselement 2 im Halteelement 1 beweglich. Das Halteelement 1 ist aber nicht translatorisch entlang des Befestigungselementes 2 bewegbar. Das Kugelgelenk ist durch ein Klemmelement oder einen Ring 12 feststellbar oder fixierbar. Der Ring weist eine Öffnung 17 auf, sowie eine biegeschwache Stelle 16, die für ein vereinfachtes Montieren des Ringes 12 in eine umlaufende Ringnut 10 des Halteelementes 1 vorgesehen sind.

**Figur 3** zeigt die Befestigungsvorrichtung mit Halteelement 1, Befestigungselement 2 und Ring 12 im nicht arretiertem Zustand im Schnitt. Eine innere Fläche oder Kontaktfläche 14 des Ringes 12 liegt in diesem Zustand mindestens annähernd symmetrisch zum Zentrum des Gelenkkopfes 3. Die Ringnut 10 ist gegenüber dem Zentrum des Gelenkkopfes 3 und gegebenenfalls auch einer Längsachse des Befestigungselementes 2 exzentrisch angeordnet. In der gezeigten Position des Ringes 12 können sich das Befestigungselement 2 und das Halteelement 1 um das Kugelgelenk zueinander bewegen.

Mittels der Öffnung 17 am Klemmelement 12 wird ein Aufweiten und ein Zusammendrücken des Ringes 12 ermöglicht. Somit kann das Klemmelement 12 zusammengedrückt und in die Ringnut 10 eingelegt werden. Durch einen Absatz 13 am Ring und eine passende Schulter 11 an der Ringnut 10 wird das Klemmelement 12 gegen Herausspringen gesichert. Anstelle des Absatzes oder zusätzlich können Ring und Ringnut auch zueinander korrespondierend konisch ausgebildet sein.

Im Bereich der Kontaktfläche 14 befinden sich ein oder mehrere Fixierelemente 15, Aufrauhungen oder makroskopische Erhebungen, die für ein Verriegeln des Gelenkkopfes 3 vorgesehen sind. Das Fixierelement 15 ist in dieser Figur nicht im Eingriff mit dem Gelenkkopf 3. Das Befestigungselement 2 ist mit einer Aufnahme für ein Werkzeug, beispielsweise einen Steckschlüssel, versehen. Die Fixierelement können auch an der Gelenkpfanne 8 oder am Gelenkkopf 3 ausgebildet sein,

Durch Verdrehen des Klemmelementes 12 verschiebt sich das Zentrum der inneren Fläche 14 des Klemmelementes 12 und arretiert somit den Gelenkkopf 3 des Befestigungselementes 2 gegenüber der Gelenkpfanne 8. Die Fixierelemente 15 schneiden sich in den Gelenkkopf 3 ein und gewähren somit eine zusätzliche Verriegelung gegen ein mögliches Lösen der Verbindung 2.

Die Masse, insbesondere Innendurchmesser und Aüssendurchmesser des Klemmelementes 12 sind vorzugsweise so gewählt, dass das Klemmelement einerseits durch Zusammendrücken in die Ringnut 10 einsetzbar ist, und andererseits das Klemmelement 12 soweit aufweitbar ist, dass der Gelenkkopf 3 durch das Klemmelement 12 hindurchgeführt werden kann.

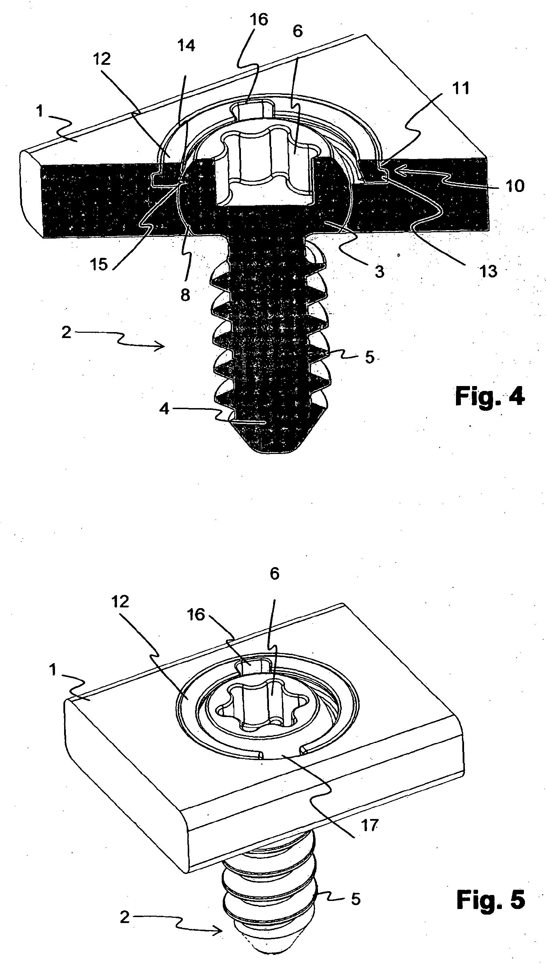

**Figur 4** zeigt die Befestigungsvorrichtung im arretierten Zustand im Schnitt. Das Befestigungselement 2 mit dem Schaft 4 und dem Gelenkkopf 3 sind weiterhin koaxial zur Gelenkpfanne 8 des Halteelementes 1 positioniert. Durch die exzentrische Anordnung der Kontaktfläche 14 des Ringes 12 bezüglich der äusseren Fläche des Ringes 12 sowie der Ringnut 10 des Halteelementes 1 wird durch die Drehung des Klemmelementes 12 das Befestigungselement 2 gegenüber dem Halteelement 1 in der Gelenkpfanne 8 arretiert. Die optionalen Fixierelemente 15 schneiden sich dabei aufgrund der exzentrischen Positionierung in den Gelenkkopf 3 des Befestigungselementes 2 ein.

**Figur 5** zeigt ebenfalls die Befestigungsvorrichtung im arretierten Zustand. In dieser Darstellung ist das Klemmelement 12 gegenüber Figur 1 und Figur 2 um ca. 60° gegen den Uhrzeigersinn verdreht. Durch Drehen im Uhrzeigersinn wird das Klemmelement 12 wieder in seine neutrale Position gebracht und löst somit die Arretierung des Befestigungselementes 2 gegenüber dem Halteelement 1. Dadurch kann eine Korrektur der Orientierung des Halteelementes 1 bezüglich des Befestigungselementes 2 erfolgen.

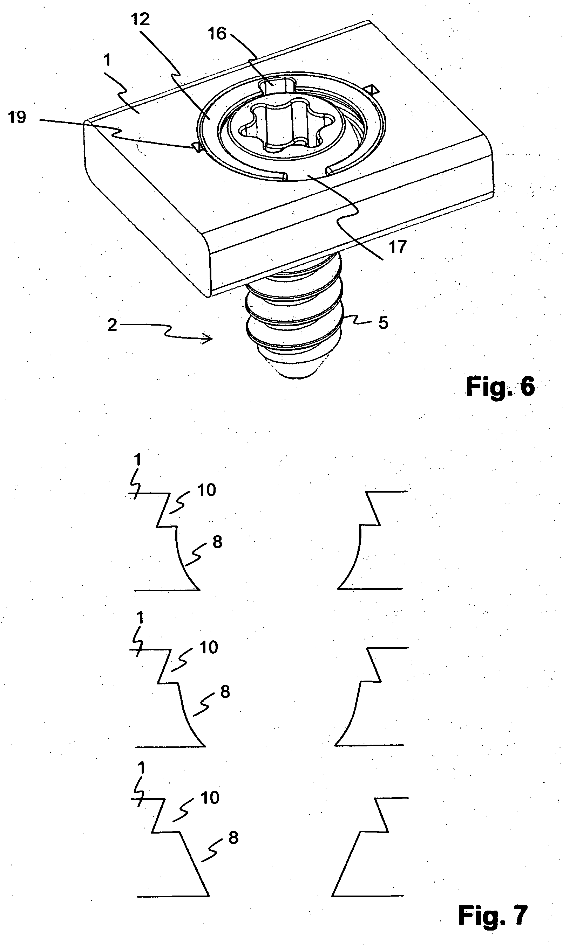

**Figur 6** zeigt eine weitere Ausführungsform einer Befestigungsvorrichtung, dessen Klemmelement 12 nach Art eines Bajonettverschlusses in der Ringnut 10 gehalten ist. Dazu sind an der Peripherie der Ringnut 10 Einschnitte 19 angeordnet, welche entsprechende Aussparungen des Ringes 12 aufnehmen. Ein Absatz 13 und eine Schulter 11 oder ein Konus sind für diese Ausführungsform nicht erforderlich.

**Figur 7** zeigt schematisch, jeweils im Querschnitt durch ein Halteelement 1, verschiedene Formen von Gelenkpfannen 8; und zwar von oben nach unten:
- eine sphärische Gelenkpfanne
- eine Gelenkpfanne 8 mit einem an die Ringnut 10 angrenzenden konischen Bereich und einem daran anschliessenden sphärischen Bereich.
- eine Gelenkpfanne 8 mit einem an die Ringnut 10 angrenzenden und bis zur Gegenseite des Haltelementes führenden konischen Bereich.
Die beiden unteren Formen, also mit einer sich zur Ringnut 10 hin öffnenden Gelenkpfanne 8, erlauben ein Einsetzen des Befestigungselementes 2 in eine Richtung, die von der Normalen zur Ausrichtung des Halteelementes 1 abweicht. Die Ringnut ist hier konisch gezeichnet, kann aber natürlich auch hier gestuft (also mit einer Schulter 11) oder kombiniert konisch/gestuft sein.

In den Figuren 1 - 7 sind jeweils Ausschnitte eines chirurgischen Halteelementes 1 gezeigt. Ein vollständiges chirurgisches Halteelement 1 weist vorzugsweise mehrere Aufnahmen für Befestigungselemente 2 auf. Bei Anwendungen an der Wirbelsäule könnten auch Ausführungsformen mit nur einem Gelenk anwendbar sein.

**BEZUGSZEICHENLISTE**

| | | | | |
|---|---|---|---|---|
| 1 | Halteelement, Platte | | 10 | Ringnut |
| 2 | Befestigungselement | | 11 | Schulter |
| 3 | Gelenkkopf | | 12 | Ring |
| 4 | Schaft | | 13 | Absatz |
| 5 | Gewinde | | 14 | Kontaktfläche |
| 6 | Aufnahme | für | 15 | Fixierelement |
| | Steckschlüssel | | 16 | biegeschwache Stelle |
| 8 | Gelenkpfanne | | 17 | Öffnung |
| 9 | Kugelfläche | | 19 | Einschnitte |

## Patentansprüche

1. Eine **Befestigungsvorrichtung für chirurgische Haltesysteme,** aufweisend ein Halteelement (1) und ein Befestigungselement (2) welche mechanisch miteinander verbindbar sind,
wobei das Halteelement (1) eine Gelenkpfanne (8) und das Befestigungselement (2) einen Gelenkkopf (3) aufweist, wobei die Gelenkpfanne (8) und der Gelenkkopf (3) zueinander korrespondierend geformt sind und ein Kugelgelenk bilden, wobei die Bewegung des Kugelgelenkes mittels eines Klemmelementes (12) arretierbar ist, **dadurch gekennzeichnet, dass** das Klemmelement ein Exzenter-Ring (12) ist, welcher beim Verdrehen den Gelenkkopf (3) bezüglich der Gelenkpfanne (8) verklemmt.

2. Die Befestigungsvorrichtung gemäss Anspruch 1, wobei das Kugelgelenk im arretierten Zustand keine translatorische Bewegung des Gelenkkopfes (3) bezüglich der Gelenkpfanne (8) zulässt.

3. Die Befestigungsvorrichtung gemäss einem der bisherigen Ansprüche, wobei das Klemmelement (12) an seiner Innenseite eine Kontaktfläche (14) aufweist, die zum Verklemmen gegen die Kugelfläche (9) des Gelenkkopfes (3) drückbar ist, wobei die Kontaktfläche (14) mindestens annähernd einen Abschnitt einer Kugelfläche bildet.

4. Die Befestigungsvorrichtung gemäss Anspruch 3, wobei die Kontaktfläche (14) und/oder die Gelenkpfanne (8) mindestens ein Fixierelement (15) aufweist, welches über den restlichen Bereich der Kontaktfläche (14) respektive der Gelenkpfanne (8) vorsteht und durch Verdrehen des Klemmelementes (12) gegen den Gelenkkopf (3) pressbar ist.

5. Die Befestigungsvorrichtung gemäss einem der bisherigen Ansprüche, wobei das mindestens eine Fixierelement (15) durch eine oder mehrere zumindest annähernd parallel zur Ebene des Klemmelementes (12) umlaufende Schneidkanten gebildet ist.

6. Die Befestigungsvorrichtung gemäss einem der bisherigen Ansprüche, wobei das Klemmelement (12) drehbar in einer Halterung im Halteelement (1) angeordnet ist, diese Halterung durch eine umlaufende Nut (10) im Halteelement (1) gebildet ist, und die Form einer Aussenseite des Klemmelementes (12) korrespondierend zur Form einer Innenseite der umlaufenden Nut (10) geformt ist, und dadurch das Klemmelement (12) am Herausfallen aus der Halterung gehindert ist.

7. Die Befestigungsvorrichtung gemäss Anspruch 6, wobei das Klemmelement (12) an seiner Aussenseite einen Absatz (13) aufweist, dessen Form mit der Form einer Schulter (11) an der Innenseite der umlaufenden Nut (10) korrespondiert.

8. Die Befestigungsvorrichtung gemäss Anspruch 6 oder 7, wobei das Klemmelement (12) an seiner Aussenseite einen Aussenkonus bildet, und die Innenseite der umlaufenden Nut (10) einen Innenkonus bildet.

9. Die Befestigungsvorrichtung gemäss einem der bisherigen Ansprüche, wobei das Klemmelement (12) eine biegeschwache Stelle (16) und optional eine Öffnung (17) aufweist und dadurch zusammendrückbar ist.

10. Die Befestigungsvorrichtung gemäss einem der bisherigen Ansprüche, wobei das Klemmelement (12) und die umlaufende Nut (10) einen Bajonettverschluss bilden.

11. **Chirurgisches Halteelement** (1), aufweisend mindestens eine Gelenkpfanne (8) mit jeweils einer anschliessenden umlaufenden Nut (10), wobei ein Klemmelement (12) in die umlaufende Nut (10) einsetzbar ist, **dadurch gekennzeichnet, dass** die umlaufende Nut (10) jeweils exzentrisch zur Achse der Gelenkpfanne (8) liegt und das Halteelement (1) mit mindestens einem in eine der Nuten (10) eingesetzten Klemmelement (12) bestückt ist, wobei das mindestens eine Klemmelement ein Exzenter-Ring (12) ist; mit welchem beim Verdrehen ein Gelenkkopf (3), welcher nicht Teil des Halteelementes (1) ist und welcher mit der Gelenkpfanne (8) ein Kugelgelenk bildet, bezüglich der Gelenkpfanne (8) verklemmbar ist.

12. Chirurgisches Halteelement, gemäss Anspruch 11, wobei ein Klemmelement (12) nach dem Einsetzen in die umlaufende Nut (10) gehalten und am Herausfallen gehindert ist.

13. Chirurgisches Halteelement, gemäss einem der Ansprüche 11 oder 12, wobei die umlaufende Nut (10) eine Schulter (11) zum Halten eines Klemmelementes (12) aufweist.

14. Chirurgisches Halteelement, gemäss einem der Ansprüche 11 bis 13, wobei die umlaufende Nut (10) einen Konus zum einrastenden Festhalten eines Klemmelementes (12) aufweist.

15. Chirurgisches Halteelement, gemäss einem der Ansprüche 11 bis 14, wobei ein äusserer Rand der umlaufenden Nut (10) mindestens einen Einschnitt (19) in axialer Richtung aufweist, welcher einen Teil eines Bajonettverschlusses mit einem Klemmelement (12) bildet.

## Claims

1. A **fastening apparatus for surgical retaining systems,** comprising a retaining element (1) and a fastening element (2), which can be mechanically connected to each other,
wherein the retaining element (1) comprises a joint socket (8) and the fastening element (2) comprises a joint head (3), wherein the joint socket (8) and the joint head (3) are shaped corresponding to each other and form a ball-and-socket joint, wherein the movement of the ball-and-socket joint can be locked by means of a clamping element (12), **characterized in that**, the clamping element is an eccentric ring (12) which, when turned, wedges the joint head (3) with respect to the joint socket (8).

2. The fastening apparatus as claimed in claim 1, wherein the ball-and-socket joint, in the locked state, does not allow any translational movement of the joint head (3) with respect to the joint socket (8).

3. The fastening apparatus as claimed in one of the preceding claims, wherein the clamping element (12) comprises, on its inner side, a contact surface (14) that can be pressed against the ball surface (9) of the joint head (3) to provide wedging, wherein the contact surface (14) at least approximately forms a portion of a ball surface.

4. The fastening apparatus as claimed in claim 3, wherein the contact surface (14) and/or the joint socket (8) comprises at least one fixing element (15), which protrudes from the rest of the area of the contact surface (14) and/or of the joint socket (8) and can be pressed against the joint head (3) by turning the clamping element (12).

5. The fastening apparatus as claimed in one of the preceding claims, wherein the at least one fixing element (15) is formed by one or more cutting edges running circumferentially and at least approximately parallel to the plane of the clamping element (12).

6. The fastening apparatus as claimed in one of the preceding claims, wherein the clamping element (12) is arranged rotatably in a mount in the retaining element (1), this mount is formed by a circumferential groove (10) in the retaining element (1), and the shape of an outer side of the clamping element (12) is shaped corresponding to the shape of an inner side of the circumferential groove (10), and in this way the clamping element (12) is prevented from falling out of the mount.

7. The fastening apparatus as claimed in claim 6, wherein the clamping element (12), on its outer side, has a flange (13), the shape of which corresponds to the shape of a shoulder (11) on the inner side of the circumferential groove (10).

8. The fastening apparatus as claimed in claim 6 or 7, wherein the clamping element (12) forms, on its outer side, an external cone, and the inner side of the circumferential groove (10) forms an internal cone.

9. The fastening apparatus as claimed in one of the preceding claims, wherein the clamping element (12) comprises a flexurally weak point (16) and optionally an opening (17) and in this way can be compressed.

10. The fastening apparatus as claimed in one of the preceding claims, wherein the clamping element (12) and the circumferential groove (10) form a bayonet catch.

11. A **surgical retaining element** (1), comprising at least one joint socket (8) with in each case an adjoining circumferential groove (10), wherein a clamping element (12) can be inserted into the circumferential groove (10), **characterized in that** the circumferential groove (10) in each case lies eccentrically with respect to the axis of the joint socket (8), and the retaining element (1) is equipped with at least one clamping element (12) inserted into one of the grooves (10), wherein the at least one clamping element is an eccentric ring (12) by means of which a joint head (3), which is not part of the retaining element (1) and which forms a ball-and-socket joint with the joint socket (8), can be wedged with respect to said joint socket (8) when the eccentric ring (12) is turned.

12. The surgical retaining element as claimed in claim 11, wherein a clamping element (12), after insertion into the circumferential groove (10), is retained and prevented from falling out.

13. The surgical retaining element as claimed in one of claims 11 or 12, wherein the circumferential groove (10) comprises a shoulder (11) for retaining a clamping element (12).

14. The surgical retaining element as claimed in one of claims 11 through 13, wherein the circumferential groove (10) comprises a taper for engaging and securing a clamping element (12).

15. The surgical retaining element as claimed in one of claims 11 through 14, wherein an outer edge of the circumferential groove (10) has at least one indent (19) in the axial direction, which indent (19) forms part of a bayonet catch with a clamping element (12).

## Revendications

1. Dispositif de fixation pour des systèmes chirurgicaux de rétention, comprenant un élément de rétention (1) et un élément de fixation (2), qui peuvent être mécaniquement reliés l'un à l'autre, l'élément de rétention (1) comprenant une emboîture (8) et l'élément de fixation (2) comprenant une tête d'articulation (3), l'emboîture (8) et la tête d'articulation (3) ayant des formes correspondant l'une à l'autre, et formant une articulation sphéroïde, le mouvement de l'articulation sphéroïde pouvant être bloqué à l'aide d'un élément de serrage (12), **caractérisé en ce que** l'élément de serrage est une bague excentrique (12), qui lors d'une torsion coince la tête d'articulation (3) par rapport à l'emboîture (8).

2. Dispositif de fixation selon la revendication 1, dans lequel l'articulation sphéroïde, à l'état bloqué, n'autorise aucun mouvement de translation de la tête d'articulation (3) par rapport à l'emboîture (8).

3. Dispositif de fixation selon l'une des revendications précédentes, dans lequel l'élément de serrage (12) comprend sur sa face intérieure une surface de contact (14), qui pour assurer un coincement peut être enfoncé contre la surface sphérique (9) de la tête d'articulation (3), la surface de contact (14) formant au moins approximativement un segment d'une surface sphérique.

4. Dispositif de fixation selon la revendication 3, dans lequel la surface de contact (14) et/ou l'emboîture (8) comprennent au moins un élément de fixation (15), qui dépasse de la zone restante, respectivement de la surface de contact (14) et de l'emboîture (8), et qui peut être appuyé contre la tête d'articulation (3) par une torsion de l'élément de serrage (12).

5. Dispositif de fixation selon l'une des revendications précédentes, dans lequel le ou les éléments de fixation (15) sont formés par une ou plusieurs arêtes, courant d'une manière approximativement parallèle au plan de l'élément de serrage (12).

6. Dispositif de fixation selon l'une des revendications précédentes, dans lequel l'élément de serrage (12) est disposé en rotation dans un dispositif de retenue dans l'élément de rétention (1), ce dispositif de retenue est formé par une rainure périphérique (10) dans l'élément de rétention (1), et la forme de la face extérieure de l'élément de serrage (12) est formée d'une manière correspondant à la forme de la face intérieure de la rainure périphérique (10), ce qui empêche que l'élément de serrage (12) ne s'échappe du dispositif de retenue.

7. Dispositif de fixation selon la revendication 6, dans lequel l'élément de serrage (12) comprend sur sa face extérieure un décrochement (13), dont la forme correspond à la forme d'un épaulement (11) sur le côté intérieur de la rainure périphérique (10).

8. Dispositif de fixation selon la revendication 6 ou 7, dans lequel l'élément de serrage (12) forme sur sa face extérieure un cône extérieur, et la face intérieure de la rainure périphérique (10) forme un cône intérieur.

9. Dispositif de fixation selon l'une des revendications précédentes, dans lequel l'élément de serrage (12) comprend une zone (16) flexible, et en option une ouverture (17), et peut de ce fait être comprimé.

10. Dispositif de fixation selon l'une des revendications précédentes, dans lequel l'élément de serrage (12) et la rainure périphérique (10) forment un emboîtement à baïonnette.

11. Elément de rétention chirurgical (1), comprenant au moins une emboîture (8), chacune comprenant une rainure périphérique contiguë (10), dans lequel un élément de serrage (12) peut être inséré dans la rainure périphérique (10), **caractérisé en ce que** chaque rainure périphérique (10) se trouve en position excentrique par rapport à l'axe de l'emboîture (8), et l'élément de rétention (1) est garni d'au moins un élément de serrage (12) inséré dans l'une des rainures (10), le ou les éléments de serrage étant des bagues excentriques (12), à l'aide d'une torsion desquelles une tête d'articulation (3) qui ne fait pas partie de l'élément de rétention (1) et qui avec l'emboîture (8) forme une articulation sphéroïde, peut être coincée par rapport à l'emboîture (8).

12. Elément de rétention chirurgical selon la revendication 11, dans lequel un élément de serrage (12) est retenu après insertion dans la rainure périphérique (10), et ne peut s'en échapper.

13. Elément de rétention chirurgical selon l'une des revendications 11 ou 12, dans lequel la rainure périphérique (10) comprend un épaulement (11) pour retenir un élément de serrage (12).

14. Elément de rétention chirurgical selon l'une des revendications 11 à 13, dans lequel la rainure périphérique (10) comprend un cône pour un maintien en place avec encliquetage d'un élément de serrage (12).

15. Elément de rétention chirurgical selon l'une des revendications 11 à 14, dans lequel un bord extérieur de la rainure périphérique (10) comprend une entaille (19) dans la direction axiale, qui forme une partie d'un emboîtement à baïonnette avec un élément de serrage (12).
